(19) **Europäisches Patentamt**

**European Patent Office**

**Office européen des brevets**

(11) **EP 1 481 985 A1**

(12) **EUROPEAN PATENT APPLICATION**

(43) Date of publication:
**01.12.2004 Bulletin 2004/49**

(51) Int Cl.⁷: **C07K 14/18**, A61K 39/29,
A61P 31/14, G01N 33/53,
G01N 33/576

(21) Application number: **04102409.2**

(22) Date of filing: **28.05.2004**

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(30) Priority: **28.05.2003 EP 03447129
28.05.2003 US 473472 P**

(71) Applicant: **INNOGENETICS N.V.**
**9052 Gent (BE)**

(72) Inventors:
• **Depla, Erik**
**9070 Destelbergen (BE)**
• **Maertens, Geert**
**8310 Brugge (BE)**
• **Bosman, Fons**
**1745 Opwijk (BE)**

(54) **Modified hepatitis C virus (HCV) NS3 for medical treatment**

(57)    The present invention relates to peptides or polypeptides comprising an HCV NS3 protein or a part thereof or a derivative of said peptide or polypeptide, HCV NS3 protein or part thereof wherein at least one cysteine is irreversibly modified. These modified HCV NS3 proteins have advantageous properties both for diagnostic and therapeutic/prophylactic applications.

**Description**

**FIELD OF THE INVENTION**

The invention relates to the fields of HCV diagnosis, HCV therapeutics and HCV prophylaxis. Specifically, the invention relates to HCV NS3 proteins and their use in the fields mentioned. More specifically, the HCV NS3 proteins are modified at their cysteine thiol-groups that are advantageously irreversibly protected.

**BACKGROUND TO THE INVENTION**

The ca. 9.6 kb single-stranded RNA genome of the HCV virus comprises 5'- and 3'-non-coding regions (NCRs) and, in between these NCRs a single long open reading frame of ca. 9 kb encoding a HCV polyprotein of ca. 3000 amino acids.

HCV polypeptides are produced by translation from the open reading frame followed by proteolytic processing of the resulting ca. 330 kDa polyprotein. Structural proteins are derived from the amino-terminal one-fourth of the polyprotein and include the capsid or Core protein (ca. 21 kDa), the E1 envelope glycoprotein (ca. 31 kDa) and the E2 envelope glycoprotein (ca. 70 kDa), previously called NS1. From the remainder of the HCV polyprotein the non-structural HCV proteins are derived which include NS2 (ca. 23 kDa), NS3 (ca. 70 kDa), NS4A (ca. 8 kDa), NS4B (ca. 27 kDa), NS5A (ca. 58 kDa) and NS5B (ca. 68 kDa) (Grakoui et al. 1993). The E2 protein can occur with or without a C-terminal fusion of the p7 protein (Shimotohno et al. 1995). Recently, an alternative open reading frame in the Core-region was found which is encoding and expressing a ca. 17 kDa protein called F (Frameshift) protein (Xu et al. 2001; Ou & Xu in US Patent Application Publication No. US2002/0076415). In the same region, ORFs for other 14-17 kDa ARFPs (Alternative Reading Frame Proteins), A1 to A4, were discovered and antibodies to at least A1, A2 and A3 were detected in sera of chronically infected patients (Walewski et al. 2001).

HCV is the major cause of non-A, non-B hepatitis worldwide. Acute infection with HCV (20% of all acute hepatitis infections) frequently leads to chronic hepatitis (70% of all chronic hepatitis cases) and end-stage cirrhosis. It is estimated that up to 20% of HCV chronic carriers may develop cirrhosis over a time period of about 20 years and that of those with cirrhosis between 1 to 4%/year is at risk to develop liver carcinoma. (Lauer & Walker 2001, Shiffman 1999). An option to increase the life-span of HCV-caused end-stage liver disease is liver transplantation (30% of all liver transplantations world-wide are due to HCV-infection).

HCV immunoassays, i.e., immunoassays capable of detecting HCV antibodies or antigens (or both), are important in the context of clinical testing as well as in the context of screening of (donated) blood and its derivatives. In a clinical test, body fluid (e.g., serum) or a solid sample of a body (e.g., liver biopsy) is diagnosed for the presence of HCV, is monitored for the course of HCV disease development and/or is monitored for the effect of a treatment in a HCV-infected individual. Large-scale screening of blood and its derivatives for the presence of HCV (as well as of, e.g., HIV and HBV) is required by regulatory authorities. As a result thereof the supply of blood or its derivatives free from pathogenic contaminants can be safeguarded.

HCV immunoassays may be divided in screening assays and confirmation assays. Preferably, the confirmation assays comprise a different set of antigens (in case of anti-HCV-antibody detection) than the set of antigens in the screening assays.

Since its discovery in 1988, the search for HCV antigens with a superior performance in immunoassays (both in terms of sensitivity and specificity) has been continuously ongoing.

HCV NS3 antigens have been described in EP 0 450 931 (C33c, spanning amino acids 1192-1457 of the HCV polyprotein), by Mori et al. 1992 (an NS3 antigen spanning amino acids 1295-1541 of the HCV polyprotein), in WO92/11370 (spanning amino acids 1007-1534 of the HCV polyprotein) and in EP 0 696 640 (D26, spanning amino acids 1207±10-1488±10 of the HCV polyprotein; and D27, spanning amino acids 1227-1528 of the HCV polyprotein). D26 was proven to have a higher specificity as compared to C33c, a higher stability as compared to the NS3 antigen spanning amino acids 1007-1534, and a higher expression level as compared to D27. The C33c NS3 antigen is a fusion protein with a N-terminal SOD fragment whereas the NS3 antigen disclosed by Mori et al. 1992 was obtained as a fusion with β-galactosidase. In general, antigens comprised in fusion proteins have the disadvantage of possible cross-reaction of antibodies in a sample with the non-HCV fusion protein and thus, to provoke false-positive test results.

WO97/12043 discloses an NS3 fragment (spanning amino acids 1027-1657 of the HCV polyprotein) with helicase activity and improved solubility.

WO91/15575 discloses a number of NS3 protease fragments.

WO91/15771 discloses a combination of a Core antigen with at least one of an envelope, NS3, NS4 or NS5 antigen. The preferred NS3 antigen herein is C33c (see above). NS3 C33c production is described in Example 1 of WO91/15771. From this latter Example it is clear that cysteine-thiol groups are not protected.

A combined NS3/4 antigen, C100-3, spanning amino acids 1569-1931 of the HCV polyprotein is disclosed in EP 0 318 216 and is obtained as a fusion protein with an N-terminal SOD fragment.

Attempts have also been made to improve conditions of immunoassays. Specifically for NS3 antigens improvements have been described in JP06074956 and EP 0 696 640, the improvement being an improved reactivity of the NS3 antigens when a reducing agent is added to the reaction mixture. WO99/54735 discloses a method of improved NS3 antigen preparation and a method for producing a solid phase immunoassay with a reduced NS3 antigen; key hereto is the reversible protection of the cysteine thiol-groups during purification of the antigen.

A HCV vaccine for prophylactic and/or therapeutic purposes may be a DNA-based vaccine, a protein- or peptide-based vaccine, or a combination of a DNA-prime protein-boost vaccination may be applied. Only vaccinations including proteins or peptides are listed below.

DNA-prime protein-boost vaccination studies have been performed in mice for Core (Hu et al. 1999) and E2 (Song et al. 2000).

Studies with protein- or peptide-based HCV vaccines, i.e. subunit HCV vaccines, are very limited and include immunization of mice with fragments of Core (Shirai et al. 1996, Hu et al. 1999), E1 (Lopez-Diaz de Cerio et al. 1999), E2 (Nakano et al. in US Patent Publication No. 2002/0119495; Houghton et al. in US Patent Application Publication No. 2002/0002272), E1/E2 or E1/E2 + Core (Drane et al. in International Patent Publication No. WO01/37869) and NS5 (Shirai et al. 1996, Uno-Furuta et al. 2001).

All of the above exploratory vaccinations were performed on rodents. Only a limited number of prophylactic and therapeutic vaccinations of primates or chimpanzees or therapeutic vaccinations of HCV-infected humans have been performed. E2 DNA-vaccinations of mice, macaques and chimpanzees were described in two studies of Forns et al. (1999, 2000). Rhesus macaques were injected with Core-expressing vaccinia virus, Core adjuvanted with LTK63 or Core adjuvanted with ISCOM in a study by Drane et al. (in International Patent Publication No. WO01/37869). Prophylactic vaccination of chimpanzees with an E1/E2 or Core/E1/E2 complex has been described in Choo et al. (1994), Houghton et al. (1995). Prophylactic and therapeutic vaccination of chimpanzees with an E1 protein has been described in WO99/67285 and WO02/055548. Interestingly, the immune responses observed in chimpanzees were also observed in HCV-infected humans and in healthy volunteers.

From the above, it will be clear that there is room for improving the diagnostic quality of HCV NS3 proteins. It will also be clear that research on the immunogenic properties of HCV NS3 has not been reported. The present invention discloses a modified HCV NS3 protein that both overcomes some of the problems encountered in its use in diagnostic assays and in addition thereto has an encouraging immunogenic potential.

## SUMMARY OF THE INVENTION

In a first aspect the current invention relates to an isolated HCV NS3 protein or a part thereof or a derivative of any thereof wherein at least one cysteine thiol groups is irreversibly modified. Further part of the invention are derivatives of an HCV NS3 protein or a part thereof or a derivative of any thereof wherein at least one cysteine thiol groups is chemically modified. In a specific embodiment thereto, at least one cysteine thiol group in said isolated peptide or polypeptide is chemically modified by irreversible alkylation.

Another aspect of the current invention relates to a composition comprising an isolated protein or part thereof or derivative of any thereof according to the invention and at least one of a pharmaceutically acceptable carrier, adjuvant or vehicle. In specific embodiments thereto, said composition is a HCV immunogenic composition, a prophylactic HCV vaccine composition or a therapeutic HCV vaccine composition. Any of said compositions may further comprise a DNA vaccine vector, in particular a HCV DNA vaccine vector.

Another aspect of the current invention relates to the use of an isolated protein or part thereof or derivative of any thereof according to the invention for the manufacture of a HCV immunogenic composition, a prophylactic HCV vaccine composition or a therapeutic HCV vaccine composition.

Further aspects of the current invention comprise the HCV immunogenic composition, an HCV vaccine composition, a prophylactic HCV vaccine composition and/or a therapeutic HCV vaccine composition according to the invention for; or alternatively comprises the use of any of said compositions for:

- inducing in a mammal a humoral response to the HCV peptides comprised in any of said compositions; and/or
- inducing in a mammal a cellular response to the HCV peptides comprised in any of said compositions, wherein said cellular response may be a CD4[+] T-cell proliferation response and/or a CD8[+] cytotoxic T-cell response and/or the increased production of cytokines; and/or
- prophylactic protection of a mammal against chronic HCV infection, wherein said HCV infection may be a homologous or a heterologous HCV infection; and/or
- therapeutically treating a chronically HCV-infected mammal, wherein said HCV may be a homologous or a heterologous HCV; and/or
- reducing liver disease in a HCV-infected mammal; and/or

- reducing liver disease in a chronic HCV-infected mammal by at least 2 points according to the overall Ishak score; and/or
- reducing serum liver enzyme activity levels in a HCV-infected mammal, wherein said liver enzyme may be, e.g., alanine aminotransferase (ALT) or gamma-glutamylpeptidase; and/or
- reducing HCV RNA levels in a HCV-infected mammal; and/or
- reducing liver fibrosis progression in a HCV-infected mammal; and/or
- reducing liver fibrosis in a HCV-infected mammal; and/or
- reducing HCV antigen levels in or presented on liver cells, wherein said HCV antigens include E2 or Core antigens.

Another aspect of the current invention relates to the use of an isolated protein or a part thereof or a derivative of any thereof according to the invention in immunoassays, to the incorporation of an isolated protein or part thereof or derivative of any thereof according to the invention in immunoassay kits or diagnostic kits, and to the use of an isolated protein or part thereof or derivative of any thereof according to the invention for the manufacture of an immunoassay kit or diagnostic kit. Immunoassays comprise immunological methods for determining the presence of antibodies to HCV in a biological sample or of antigens of HCV in a biological sample or of HCV virus in a biological sample, or for diagnosing HCV infection. Diagnostic kits or immunoassay kits comprise kits for determining the presence of antibodies to HCV in a biological sample or of antigens of HCV in a biological sample or of HCV virus in a biological sample, or for diagnosing HCV infection.

A first general embodiment in relation to immunoassays comprises a method for determining the presence of antibodies to HCV, in particular to HCV NS3, in a biological sample comprising the step of detecting said antibodies to an isolated protein or part thereof or derivative of any thereof according to the invention.

A second general embodiment in relation to immunoassays comprises a method for determining the presence of HCV NS3 antigens in a biological sample comprising the step of detecting said HCV NS3 antigens with an antibody to said HCV NS3 antigens in the presence of an isolated protein or part thereof or derivative of any thereof according to the invention as competitor of binding of said HCV NS3 antigens to said antibody.

The invention further relates to a method for producing the HCV NS3 protein or part thereof or derivative of any thereof according to the invention wherein said method comprises the steps of:

(i) obtaining an HCV NS3 protein or part thereof by means of recombinant expression or chemical synthesis;
(ii) irreversibly modifying at least one cysteine thiol group in the HCV NS3 protein or part thereof obtained in (i);
(iii) purifying the HCV NS3 protein or part thereof of (ii).

## FIGURE LEGENDS

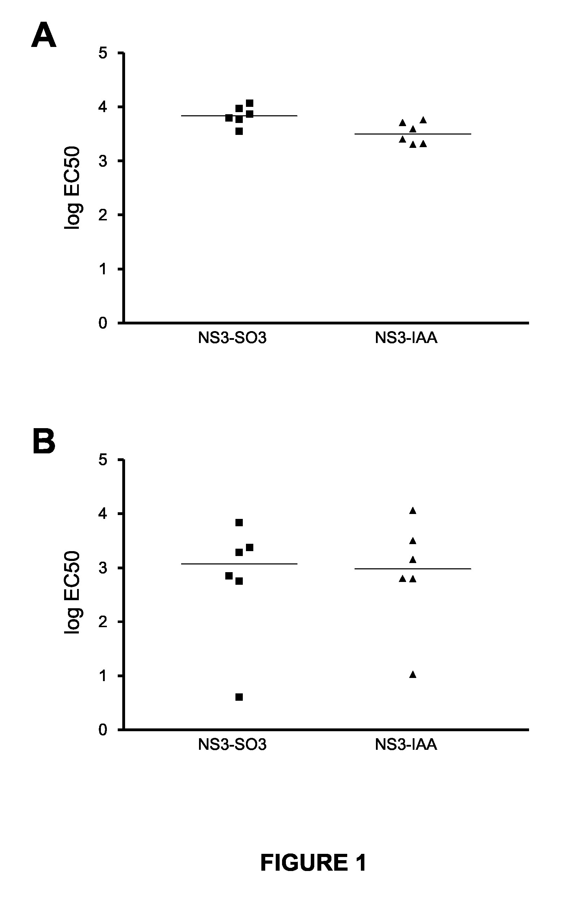

**Figure 1.** Log EC50 values of antibody titers induced in mice upon immunization with sulfonated (A) or alkylated (B) HCV NS3. The ELISA was performed with either alkylated (IAA) or desulfonated (SO3) HCV NS3 as coated reagent as indicated in the X-axis. The horizontal lines represent mean values. The underlying experiment is outlined in Example 3 herein.

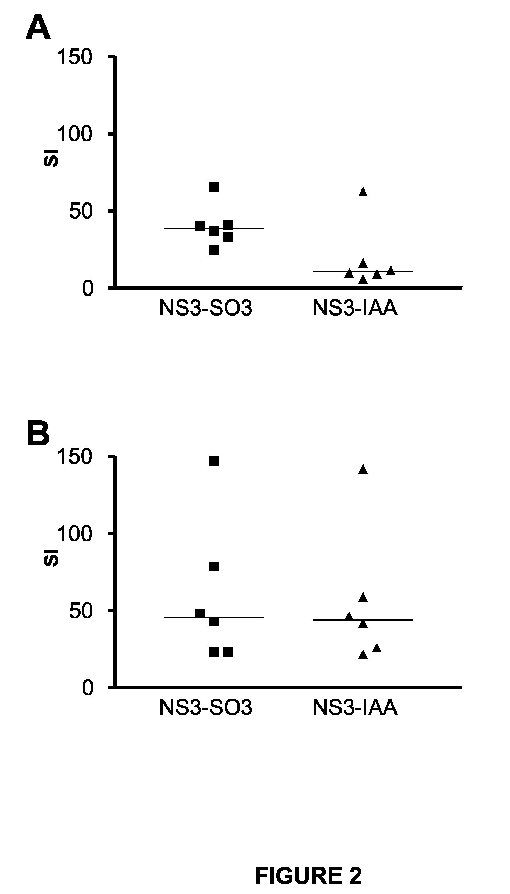

**Figure 2.** Stimulation Index (SI) values, reflecting the cellular immune response induced in mice upon immunization with sulfonated (A) or alkylated (B) HCV NS3. The in vitro restimulation was performed with either alkylated (IAA) or sulfonated (SO3) HCV NS3 as indicated in the X-axis. The horizontal lines represent mean values. The underlying experiment is outlined in Example 3 herein.

## DETAILED DESCRIPTION OF THE INVENTION

The present invention relates to the diagnostic and immunogenic properties of HCV NS3 proteins wherein at least one cysteine has been reversibly or irreversibly modified. Cysteines in HCV NS3 have more specifically been sulfonated or alkylated yielding NS3-SO3 or NS3-IAA, respectively.

In WO91/15771, the C33c antigen yielded 60% (chronic HCV carrier serum samples only) or 53% (all HCV carrier serum samples) HCV-positive scores under conditions where no reducing agent was present in the assay (see Table 1 in WO91/15771). From the healthy donor serum samples 19% tested HCV-positive, i.e. false positive, with the C33c antigen under the same conditions (see Table 2 in WO91/15771). It is known that sensitivity of C33c (NS3 antigen spanning amino acids 1182-1457 of the HCV polyprotein) is increased when a reducing agent is added to the assay medium (see, e.g., JP06074956). Of all HCV carrier serum samples tested in the present invention (see Example 2 and Table 1 herein) 100% are HCV-positive, both in an ELISA with an NS3-IAA protein and a reduced NS3-SO3 protein according to the present invention. Furthermore, none of the healthy donor serum samples tested HCV-positive with either of said NS3 proteins (see Example 2 and Table 1 herein). These results are thus clearly superior to the results with the C33c antigen as described in WO91/15771. Surprising is further that the NS3-IAA protein of the present invention is an equally good antigen as compared to the reduced NS3-SO3 protein. The additional advantages of using NS3-IAA over NS3-SO3 in diagnostic assays relate to the fact that a reducing agent can be omitted during production of such diagnostic assays as well as during the use of such diagnostic assays. Thus potential toxic effects of reducing agents are eliminated as well as the unpleasant smell of such agents. For example, 2-mercaptoethanol is toxic by inhalation, ingestion and through skin contact, is a severe eye irritant and is readily absorbed through the skin (info from a material safety data sheet). Another reducing agent, namely dithiothreitol (DTT) is known to have the following potential adverse health effects (info from a material safety data sheet): (i) upon inhalation: causes irritation to the respiratory tract; symptoms may include coughing, shortness of breath; can cause nausea, headache and vomiting; exposure may result in blood in urine, difficulty breathing, irregular heartbeat, anaemia, weakness, drunkenness, bluish skin color, lung congestion, kidney damage, paralysis, convulsions, unconsciousness and coma. Thiols may cause central nervous system depression (CNS); (ii) upon ingestion: exposure can cause nausea, headache, vomiting, diarrhea, weakness, drunkenness, restlessness, bluish skin color, paralysis and coma; (iii) upon skin contact: causes irritation to skin; symptoms include redness, itching, and pain; may be absorbed through the skin; may cause dermatitis; (iv) upon eye contact: causes irritation, redness, and pain.

The immunogenic properties of NS3-IAA have not been explored until the present invention. It is moreover herein shown for the first time that due to or despite of the modifications present in the NS3-IAA protein, such a protein is a surprisingly good immunogen (see Example 3 and Figures 1 and 2 herein), a prerequisite for its use in therapeutic and/or prophylactic applications.

A further advantage of NS3-IAA over NS3-SO3, both for diagnostic and therapeutic/prophylactic applications is the higher chemical stability of NS3-IAA over NS3-S03.

In a first aspect the current invention relates to an isolated HCV NS3 protein or a part thereof or derivative of any thereof wherein at least one of cysteine thiol groups is irreversibly modified. Further part of the invention are derivatives of an HCV NS3 protein or a part thereof or a derivative of any thereof wherein at least one cysteine thiol groups is chemically modified. In a specific embodiment thereto, at least one cysteine thiol group in said isolated peptide or polypeptide is chemically modified by irreversible alkylation.

The terms peptide, polypeptide and protein are used interchangeably herein.

A derivative of a protein of the invention, e.g. an HCV NS3 protein or a part thereof, is meant to include proteins comprising derivatized amino acids (e.g., conjugated with biotin or digoxigenin) or non-natural amino acids, HCV NS3 proteins comprising insertions, deletions or substitutions (relative to a naturally occurring HCV NS3 sequence) of one or more amino acids, as well as fusion proteins. A derivatized amino acid includes a derivatized cysteine wherein the derivatization is a modification of the thiol group and/or another modification. Fusion proteins may be formed between two distinct HCV peptides or between an HCV NS3 peptide and another peptide or protein such as a B-cell epitope, a T-cell epitope, a CTL epitope or a cytokine. Other peptide or protein fusion partners include bovine serum album, keyhole limpet hemocyanin, soybean or horseradish peroxidase, beta-galactosidase, luciferase, alkaline phosphatase, glutathione S-transferase or dihydrofolate reductase or heterologous epitopes such as (histidine)$_6$-tag, protein A, maltose-binding protein, Tag•100 epitope, c-myc epitope, FLAG®-epitope, lacZ, CMP (calmodulin-binding peptide), HA epitope, protein C epitope or VSV epitope. Other proteins include histones, single-strand binding protein (ssB) and native and engineered fluorescent proteins such as green-, red-, blue-, yellow-, cyan-fluorescent proteins.

The HCV NS3 protein corresponds to the HCV polyprotein region spanning amino acids 1027-1657. It is to be understood that these endpoints are approximations. The mentioned endpoints are not absolute as they may vary, e.g., due to insertions/deletions in an upstream part of the HCV polyprotein or in the HCV NS3 region itself. Such insertions/deletions are known to be present as is apparent when HCV polyprotein sequences of different genotypes are compared. With a part of an HCV NS3 protein is meant any part that comprises at least one cysteine residue, alternatively said part comprises 2, 3, 4, 5, 6, 7, 8, 9, 10, 11, 12, 13, 14, 15, 16, 17, 18, 19, 20 or up to all cysteine (naturally occurring and/or introduced, see further) residues of an HCV NS3 protein, the current upper limit of genotype-, subtype- or isolate-dependent number of cysteine residues in a naturally occurring HCV NS3 protein being 21. Said cysteine residue may be either a naturally occurring cysteine residue or a non-naturally occurring cysteine residue introduced, e.g., by genetic engineering or during synthetic protein manufacturing. Preferably, said part of HCV NS3 comprises at least one HCV NS3 epitope (B-cell epitope or T-cell epitope).

In a specific embodiment, the peptide or polypeptide of the invention or a derivative thereof is comprising the HCV NS3 peptide spanning amino acids 1188 to 1468 of the HCV polyprotein. The peptide or polypeptide or derivative thereof may further comprise the HCV NS3 peptide spanning amino acids 1071 to 1084 of the HCV polyprotein region or parts thereof, such as amino acids 1073 to 1081 of the HCV polyprotein region. Said peptide or polypeptide or derivative thereof may also comprise the HCV NS3 peptide spanning amino acids 1188 to 1468 and the HCV NS3 peptide spanning amino acids 1071 to 1084 or parts thereof. In a further specific embodiment, the peptide or polypeptide of the invention or a derivative thereof is comprising the HCV NS3 peptide spanning amino acids 1188 to 1468 defined by SEQ ID NO:2. Said peptide or polypeptide or derivative thereof may further comprise the HCV NS3 peptide spanning amino acids 1071 to 1084 defined by SEQ ID NO:3 or parts thereof, such as amino acids 1073 to 1081, defined by, e. g., SEQ ID NO:4. Said peptide or polypeptide or derivative thereof may also comprise the HCV NS3 peptides defined by SEQ ID NO:2 and by SEQ ID NO:3 or SEQ ID NO:4. In particular, said peptide or polypeptide of the invention may be defined by SEQ ID NO:1. For recombinant expression purposes, an amino-terminal methionine may be included in the peptide or polypeptide of the invention.

A further aspect of the invention relates to a method for producing the HCV NS3 protein or part thereof or derivative of any thereof according to the invention wherein said method comprises the steps of:

(i) obtaining an HCV NS3 protein or part thereof by means of recombinant expression or chemical synthesis;
(ii) irreversibly modifying at least one cysteine thiol group in the HCV NS3 protein or part thereof obtained in (i);
(iii) purifying the HCV NS3 protein or part thereof of (ii).

Any of the proteins, parts thereof or derivatives of any thereof according to the present invention may be of synthetic origin, i.e. synthesized by applying organic chemistry, or of recombinant origin. HCV peptides may be produced by expression in, e.g., mammalian or insect cells infected with recombinant viruses, yeast cells or bacterial cells.

More particularly, said mammalian cells include HeLa cells, Vero cells, RK13 cells, MRC-5 cells, Chinese hamster ovary (CHO) cells, Baby hamster kidney (BHK) cells and PK15 cells. More particularly, said insect cells include cells of *Spodoptera frugiperda,* such as Sf9 cells. More particularly, said recombinant viruses include recombinant vaccinia viruses, recombinant adenoviruses, recombinant baculoviruses, recombinant canary pox viruses, recombinant Semliki Forest viruses, recombinant alphaviruses, recombinant Ankara Modified viruses and recombinant avipox viruses. More particularly, said yeast cells include cells of *Saccharomyces,* such as *Saccharomyces cerevisiae, Saccharomyces kluyveri,* or *Saccharomyces uvarum, Schizosaccharomyces,* such as *Schizosaccharomyces pombe, Kluyveromyces,* such as *Kluyveromyces lactis, Yarrowia,* such as *Yarrowia lipolytica, Hansenula,* such as *Hansenula polymorpha, Pichia,* such as *Pichia pastoris, Aspergillus species, Neurospora,* such as *Neurospora crassa,* or *Schwanniomyces,* such as *Schwanniomyces occidentalis,* or mutant cells derived from any thereof. More specifically, the HCV peptide or part thereof according to the invention is the product of expression in a *Hansenula* cell. More particularly, said bacterial cells include cells of *Escherichia coli* or *Streptomyces* species.

An epitope is referring to a structure capable of binding to and/or activating a cell involved in eliciting an immune response to said structure. Epitopes thus include epitopes of B-cells, T-cells, T-helper cells and CTLs. Epitopes include conformational epitopes and linear epitopes. Peptide- or protein-epitopes comprise peptides or parts of peptides or proteins capable of binding to, e.g., T-cell receptors, B-cell receptors, antibodies or MHC molecules. The size of linear peptide- or protein-epitopes can be limited to a few, e.g. 4, 5, 6, 7, 8, 9, 10, 11, 12, 13, 14 or 15 amino acids. An epitope is antigenic but not always immunogenic.

A T-cell stimulating epitope refers to an epitope capable of stimulating T-cells, T-helper cells or CTL-cells. A T-helper cell stimulating epitope may be selected by monitoring the lymphoproliferative response, also referred to as $CD4^+$ T-cell proliferation response, towards (potential antigenic) polypeptides containing in their amino acid sequence a (putative) T-cell stimulating epitope. Said lymphoproliferative response may be measured by either a T-helper assay comprising *in vitro* stimulation of peripheral blood mononuclear cells (PBMCs) from patient sera with varying concentrations of peptides to be tested for T-cell stimulating activity and counting the amount of radiolabelled thymidine taken up by the PBMCs. Proliferation is considered positive when the stimulation index (mean cpm of antigen-stimulated cultures/mean cpm of controle cultures) is more than 1, preferably more than 2, most preferably more than 3. A CTL-stimulating epitope may be selected by means of a cytotoxic T-lymphocyte or cytotoxic T-cell (CTL) assay measuring the lytic activity of cytotoxic cells, also referred to as $CD8^+$ CTL response, using $^{51}$Cr release. Cell-mediated responses may also be assessed by measuring cytokine production, e.g., by an ELISpot assay (see for instance Fujihashi et al. 1993). Characteristic for a Th1-like response is the production/secretion of, e.g., IL-2 and/or IFN-$\gamma$. Characteristic for a Th2-like response is the production/secretion of, e.g., IL-4.

In the protein, part thereof or derivative of any thereof comprising at least one cysteine residue, the cysteine thiol-group(s) can be irreversibly protected by chemical means. "Irreversible protection" or "irreversible blocking" by chemical means refers to alkylation, preferably alkylation of the HCV NS3 proteins by means of alkylating agents, such as, for example, active halogens, ethylenimine or N-(iodoethyl)trifluoro-acetamide. In this respect, it is to be understood that alkylation of cysteine thiol-groups refers to the replacement of the thiol-hydrogen by $(CH_2)_nR$, in which n is 0, 1, 2, 3 or 4 and R= H, COOH, $NH_2$, $CONH_2$, phenyl, or any derivative thereof. Alkylation can be performed by any method known in the art, such as, for example, active halogens $X(CH_2)_nR$ in which X is a halogen such as I, Br, Cl or F. Examples of active halogens are methyliodide, iodoacetic acid, iodoacetamide, and 2-bromoethylamine. Other methods of alkylation include the use of NEM (N-ethylmaleimide) or Biotin-NEM, a mixture thereof, or ethylenimine or N-(iodoethyl) trifluoroacetamide both resulting in substitution of -H by $-CH_2-CH_2-NH_2$ (Hermanson, G. T. 1996). The term "alkylating agents" as used herein refers to compounds which are able to perform alkylation as described herein.

It is further understood that in the purification procedure, the cysteine thiol-groups of the HCV proteins, i.e. HCV NS3 proteins, or the parts thereof or the derivatives of any thereof of the present invention can be reversibly protected. The purpose of reversible protection is to stabilize the HCV protein or part thereof or derivative of any thereof. Especially, after reversible protection the sulfur-containing functional group (e.g. thiols and disulfides) is retained in a non-reactive condition. The sulfur-containing functional group is thus unable to react with other compounds, e.g. have lost their tendency of forming or exchanging disulfide bonds, such as, for example

$$R_1\text{-SH} + R_2\text{-SH} \text{ ---X---> } R_1\text{-S-S-}R_2 \text{ ;}$$

$$R_1\text{-S-S-}R_2 + R_3\text{-SH} \text{ ---X---> } R_1\text{-S-S-}R_3 + R_2\text{-SH;}$$

$$R_1\text{-S-S-}R_2 + R_3\text{-S-S-}R_4 \text{ ---X---> } R_1\text{-S-S-}R_3 + R_2\text{-S-S-R4.}$$

The described reactions between thiols and/or disulfide residues are not limited to intermolecular processes, but may also occur intramolecularly.

The term "reversible protection" or "reversible blocking" as used herein contemplates covalently binding of modification agents to the cysteine thiol-groups, as well as manipulating the environment of the HCV protein such, that the redox state of the cysteine thiol-groups remains unaffected throughout subsequent steps of the purification procedure (shielding). Reversible protection of the cysteine thiol-groups can be carried out chemically or enzymatically.

The term "reversible protection by enzymatical means" as used herein contemplates reversible protection mediated by enzymes, such as for example acyl-transferases, e.g. acyl-transferases that are involved in catalysing thio-esterification, such as palmitoyl acyltransferase (see below).

The term "reversible protection by chemical means" as used herein contemplates reversible protection:

1. by modification agents that reversibly modify cysteinyls such as for example by sulfonation and thio-esterification; Sulfonation is a reaction where thiol or cysteines involved in disulfide bridges are modified to $S$-sulfonate: RSH $\rightarrow$ RS-SO$_3$- (Darbre, A. 1986) or RS-SR$\rightarrow$ 2 RS-SO$_3$-(sulfitolysis; (Kumar, N. et al. 1986)). Reagents for sulfonation are e.g. Na$_2$SO$_3$, or sodium tetrathionate. The latter reagents for sulfonation are used in a concentration of 10-200 mM, and more preferentially in a concentration of 50-200 mM. Optionally sulfonation can be performed in the presence of a catalysator such as, for example Cu$^{2+}$ (100 µM-1 mM) or cysteine (1-10 mM).

The reaction can be performed under protein denaturing as well as native conditions (Kumar, N. et al. 1985, Kumar, N. et al. 1986).

Thioester bond formation, or thio-esterification is characterised by:

$$RSH + R'COX \rightarrow RS\text{-}COR'$$

in which X is preferentially a halogenide in the compound R'CO-X.

2. by modification agents that reversibly modify the cysteinyls of the present invention such as, for example, by heavy metals, in particular Zn$^{2+}$, Cd$^{2+}$, mono-, dithio- and disulfide-compounds (e.g. aryl- and alkylmethanethiosulfonate, dithiopyridine, dithiomorpholine, dihydrolipoamide, Ellmann reagent, aldrothiol™ (Aldrich) (Rein, A. et al. 1996), dithiocarbamates), or thiolation agents (e.g. gluthathion, N-Acetyl cysteine, cysteineamine). Dithiocarbamate comprise a broad class of molecules possessing an R$_1$R$_2$NC(S)SR$_3$ functional group, which gives them the ability to react with sulfydryl groups. Thiol containing compounds are preferentially used in a concentration of 0.1-50 mM, more preferentially in a concentration of 1-50 mM, and even more preferentially in a concentration of 10-50 mM;

3. by the presence of modification agents that preserve the thiol status (stabilise), in particular antioxidantia, such as for example DTT, dihydroascorbate, vitamins and derivates, mannitol, amino acids, peptides and derivates (e. g. histidine, ergothioneine, carnosine, methionine), gallates, hydroxyanisole, hydoxytoluene, hydroquinon, hydroxymethylphenol and their derivates in concentration range of 10 µM-10 mM, more preferentially in a concentration of 1-10 mM;

4. by thiol stabilising conditions such as, for example, (i) cofactors as metal ions (Zn$^{2+}$, Mg$^{2+}$), ATP, (ii) pH control (e.g. for proteins in most cases pH ~5 or pH is preferentially thiol pK$_a$ -2; e.g. for peptides purified by Reversed Phase Chromatography at pH ~2).

Combinations of reversible protection as described in (1), (2), (3) and (4) may result in similarly pure and refolded HCV proteins. In effect, combination compounds can be used, such as, for example Z103 (Zn carnosine), preferentially in a concentration of 1-10 mM. It should be clear that reversible protection also refers to, besides the modification groups or shielding described above, any cysteinyl protection method which may be reversed enzymatically or chemically, without disrupting the peptide backbone. In this respect, the present invention specifically refers to peptides prepared by classical chemical synthesis (see above), in which, for example, thioester bounds are cleaved by thioesterase, basic buffer conditions (Beekman, N. J. et al. 1997) or by hydroxylamine treatment (Vingerhoeds, M. H. et al. 1996).

Reversible protection may also be used to increase the solubilisation and extraction of peptides (Pomroy, N. C. and Deber, C. M. 1998).

The reversible protection and thiol stabilizing compounds may be presented under a monomeric, polymeric or liposomic form.

The removal of the reversibly protection state of the cysteine residues can chemically or enzymatically accomplished by e.g.:

- a reductant, in particular DTT, DTE, 2-mercaptoethanol, dithionite, $SnCl_2$, sodium borohydride, hydroxylamine, TCEP, in particular in a concentration of 1-200 mM, more preferentially in a concentration of 50-200 mM;
- removal of the thiol stabilising conditions or agents by e.g. pH increase;
- enzymes, in particular thioesterases, glutaredoxine, thioredoxine, in particular in a concentration of 0.01-5 $\mu$M, even more particular in a concentration range of 0.1-5 $\mu$M.;
- combinations of the above described chemical and/or enzymatical conditions.

The removal of the reversibly protection state of the cysteine residues can be carried out *in vitro* or *in vivo,* e. g. in a cell or in an individual.

It will be appreciated that in the purification procedure, the cysteine residues may or may not be irreversibly blocked, or replaced by any reversible modification agent, as listed above. Reversibly blocked cysteines in a protein may be converted to irreversibly blocked cysteines.

A reductant according to the present invention is any agent which achieves reduction of the sulfur in cysteine residues, e.g. "S-S" disulfide bridges, desulfonation of the cysteine residue ($RS\text{-}SO_3^- \rightarrow RSH$). An antioxidant is any reagent which preserves the thiol status or minimises "S-S" formation and/or exchanges. Reduction of the "S-S" disulfide bridges is a chemical reaction whereby the disulfides are reduced to thiol (-SH). "S-S" Reduction can be obtained by (1) enzymatic cascade pathways or by (2) reducing compounds. Enzymes like thioredoxin, glutaredoxin are known to be involved in the in vivo reduction of disulfides and have also been shown to be effective in reducing "S-S" bridges in vitro. Disulfide bonds are rapidly cleaved by reduced thioredoxin at pH 7.0, with an apparent second order rate that is around $10^4$ times larger than the corresponding rate constant for the reaction with DTT. The reduction kinetic can be dramatically increased by preincubation the protein solution with 1 mM DTT or dihydrolipoamide (Holmgren, A. 1979). Thiol compounds able to reduce protein disulfide bridges are for instance Dithiothreitol (DTT), Dithioerythritol (DTE), $\beta$-mercaptoethanol, thiocarbamates, bis(2-mercaptoethyl) sulfone and N,N'-bis(mercaptoacetyl)hydrazine, and sodium-dithionite. Reducing agents without thiol groups like ascorbate or stannous chloride ($SnCl_2$), which have been shown to be very useful in the reduction of disulfide bridges in monoclonal antibodies (Thakur, M. L. et al. 1991), may also be used for the reduction of HCV proteins. In addition, changes in pH values may influence the redox status of HCV proteins. Sodium borohydride treatment has been shown to be effective for the reduction of disulfide bridges in peptides (Gailit, J. 1993). Tris (2-carboxyethyl)phosphine (TCEP) is able to reduce disulfides at low pH (Bums, J. et al. 1991). Selenol catalyses the reduction of disulfide to thiols when DTT or sodium borohydride is used as reductant. Selenocysteamine, a commercially available diselenide, was used as precursor of the catalyst (Singh, R. and Kats, L. 1995).

Another aspect of the current invention relates to a composition comprising an isolated protein or part thereof or derivative of any thereof according to the invention (see first aspect of the invention) and at least one of a pharmaceutically acceptable carrier, adjuvant or vehicle. In specific embodiments thereto, said composition is a HCV immunogenic composition, a prophylactic HCV vaccine composition or a therapeutic HCV vaccine composition. In particular a HCV immunogenic composition, a prophylactic HCV vaccine composition or a therapeutic HCV vaccine composition comprises an effective amount of an isolated protein or part thereof or derivative of any thereof according to the invention. Any of the listed compositions may further comprise a DNA vaccine vector, e.g., a HCV DNA vaccine vector.

The term "immunogenic" refers to the ability of a protein or a substance to produce at least one element of an immune response. The immune response is the total response of the body of an animal to the introduction of an antigen and comprises multiple elements including antibody formation (humoral response or humoral immunity), cellular immunity, hypersensitivity, or immunological tolerance. Cellular immunity refers to cellular responses elicited by an antigen and include a T-helper cell- and/or CTL-response and/or stimulated cytokine production. The term "antigen" refers to the ability of a peptide, protein or other substance to be antigenic or immunogenic. An antigen is understood to comprise at least one epitope.

"Antigenic" refers to the capability of a protein or substance to be recognized by an elicited humoral and/or cellular immune response. Typically, the antigenic quality of a protein or substance is determined by in vitro assays. For humoral responses, a protein or substance can be referred to as antigenic in case the protein or substance is recognized by elicited antibodies in e.g. an ELISA, western-blot, RIA, immunoprecipitation assay or any similar assay in which the protein or substance is allowed to be recognized by an elicited antibody and in which such a recognition can be measured by, e.g., a colorometric, fluorometric or radioactive detection, or formation of a precipitate. For cellular response, a protein or substance can be referred to as antigenic in case the protein or substance is recognized by an elicited T-cell response in e.g. an T-cell proliferation assay, a $^{51}$Cr-release assay, a cytokine secretion assay or alike in which the protein or substance is incubated in the presence of T-cells drawn from an individual in which immune response have been elicited and in which a recognition by the T-cell is measured by, e.g., a proliferative response, a cell lysis response, a cytokine secretion. An antigenic protein or substance may be immunogenic in se but may also require additional structures to be rendered immunogenic.

An "immunogenic composition" is a composition referred to as comprising an antigen capable of eliciting at least one element of the immune response against the antigen comprised in said composition when said composition is introduced into the body of an animal capable of raising an immune response. An immunogenic composition may comprise more than one antigen, i.e., a plurality of antigens, e.g. 2, 3, 4, 5, 6, 7, 8, 9, 10 or more, e.g., up to 15, 20, 25, 30, 40 or 50 or more distinct antigens. In particular, the immunogenic composition of the invention is an HCV immunogenic composition wherein the antigen or plurality of antigens are peptide(s) or polypeptide(s) or protein(s) of the invention comprising an HCV NS3 protein or a part thereof or a derivative of any thereof modified as described herein. Said plurality of antigens may comprise a combination of HCV NS3 proteins or parts thereof or derivatives of any thereof derived from different HCV genotypes and/or subtypes and/or isolates.

A "vaccine composition" is an immunogenic composition capable of eliciting an immune response sufficiently broad and vigorous to provoke one or both of:

- a stabilizing effect on the multiplication of a pathogen already present in a host and against which the vaccine composition is targeted; and
- an effect increasing the rate at which a pathogen newly introduced in a host, after immunization with a vaccine composition targeted against said pathogen, is resolved from said host.

A vaccine composition may also provoke an immune response broad and strong enough to exert a negative effect on the survival of a pathogen already present in a host or broad and strong enough to prevent an immunized host from developing disease symptoms caused by a newly introduced pathogen. In particular the vaccine composition of the invention is a HCV vaccine composition wherein the pathogen is HCV.

An "effective amount" of an antigen in a vaccine composition is referred to as an amount of antigen required and sufficient to elicit an immune response. It will be clear to the skilled artisan that the immune response sufficiently broad and vigorous to provoke the effects envisaged by the vaccine composition may require successive (in time) immunizations with the vaccine composition as part of a vaccination scheme or vaccination schedule. The "effective amount" may vary depending on the health and physical condition of the individual to be treated, the taxonomic group of the individual to be treated (e.g. human, non-human primate, primate, etc.), the capacity of the individual's immune system to mount an effective immune response, the degree of protection desired, the formulation of the vaccine, the treating doctor's assessment, the strain of the infecting pathogen and other relevant factors. It is expected that the amount will fall in a relatively broad range that can be determined through routine trials. Usually, the amount will vary from 0.01 to 1000 µg/dose, more particularly from 0.1 to 100 µg/dose. Dosage treatment may be a single dose schedule or a multiple dose schedule. The vaccine may be administered in conjunction with other immunoregulatory agents.

A "prophylactic vaccine composition" is a vaccine composition providing protective immunity, i.e., an immunity preventing development of disease upon challenge of the host immunized with the prophylactic vaccine composition. In particular for HCV, a prophylactic HCV vaccine composition is to be understood as a vaccine composition capable of providing protective immunity helping to resolve a challenge HCV infection rapidly and/or preventing a challenge HCV infection to proceed to a chronic infection. Accelerated HCV viral clearance or accelerated control of HCV challenge infection is thus envisaged by vaccination with a prophylactic HCV composition according to the invention.

A "prophylactically effective amount" of an antigen in a prophylactic vaccine composition is referred to as an amount of antigen required and sufficient to elicit an immune response enabling the development of protective immunity. It will be clear to the skilled artisan that the immune response sufficiently broad and vigorous to provoke the effects envisaged by the prophylactic vaccine composition may need require successive (in time) immunizations with the prophylactic vaccine composition (see also "effective amount").

A "therapeutic vaccine composition" is a vaccine composition providing a curative immune response, i.e., an immune response capable of effectuating a reversion, or at least capable of effectuating halting, of disease symptoms associated with an already established pathogen infection. In particular for HCV, a therapeutic HCV vaccine composition is to be understood as a vaccine compositions capable of reducing serum liver enzyme, e.g., alanine aminotransferase (ALT) or $\gamma$-glutamylpeptidase ($\gamma$-GT), activity levels in the blood and/or of reducing HCV RNA levels and/or of reducing liver disease and/or of reducing liver fibrosis and/or of reducing liver fibrosis progression and/or reducing HCV antigen levels in or presented on liver cells.

A "therapeutically effective amount" of an antigen in a therapeutic vaccine composition is referred to as an amount of antigen required and sufficient to elicit an immune response enabling the development of a curative immune response. It will be clear to the skilled artisan that the antigenic or immunogenic response sufficiently broad and vigorous to provoke the effects envisaged by the therapeutic vaccine composition may need require successive (in time) immunizations with the therapeutic vaccine composition (see also "effective amount").

The HCV immunogenic composition, HCV vaccine composition, prophylactic HCV vaccine composition and/or therapeutic HCV vaccine composition of the invention comprises an HCV NS3 protein or a part thereof or a derivative of any thereof modified as described herein.

Another aspect of the current invention relates to the use of an isolated protein or part thereof or derivative of any thereof according to the invention for the manufacture of a HCV immunogenic composition, a prophylactic HCV vaccine composition or a therapeutic HCV vaccine composition.

Further aspects of the current invention comprise the HCV immunogenic composition, an HCV vaccine composition, a prophylactic HCV vaccine composition and/or a therapeutic HCV vaccine composition according to the invention for; or alternatively comprises the use of said composition for:

- inducing in a mammal a humoral response to the HCV peptides comprised in any of said compositions; and/or
- inducing in a mammal a cellular response to the HCV peptides comprised in any of said compositions, wherein said cellular response may be a CD4$^+$ T-cell proliferation response and/or a CD8$^+$ cytotoxic T-cell response and/or the increased production of cytokines; and/or
- prophylactic protection of a mammal against chronic HCV infection, wherein said HCV infection may be a homologous or a heterologous HCV infection; and/or
- therapeutically treating a chronically HCV-infected mammal, wherein said HCV may be a homologous or a heterologous HCV; and/or
- reducing liver disease in a HCV-infected mammal; and/or
- reducing liver disease in a chronic HCV-infected mammal by at least 2 points according to the overall Ishak score; and/or
- reducing serum liver enzyme activity levels in a HCV-infected mammal, wherein said liver enzyme may be, e.g., alanine aminotransferase (ALT) or gamma-glutamylpeptidase; and/or
- reducing HCV RNA levels in a HCV-infected mammal; and/or
- reducing liver fibrosis progression in a HCV-infected mammal; and/or
- reducing liver fibrosis in a HCV-infected mammal; and/or
- reducing HCV antigen levels in or presented on liver cells, wherein said HCV antigens include E2 or Core antigens.

Said mammal obviously may be a human. In particular, the uses according to the invention are methods for obtaining at least one of the recited effects, with said methods comprising administering any of said compositions to a mammal or a human. The recited effects may be obtained in combination with a DNA vaccine or with a DNA vector or DNA vaccine vector capable of expressing or effectuating expression of one or more antigens. A DNA vaccine, DNA vector or DNA vaccine vector may be a HCV DNA vaccine, HCV DNA vector or HCV DNA vaccine vector (see further).

With "prophylactic protection against infection by a homologous HCV" is meant that protection is obtained against a challenge HCV virus of exactly the same genotype, subtype or isolate as compared to the HCV genotype, subtype or isolate from which the HCV antigen or HCV antigens are derived. A composition may for example comprise a peptide or polypeptide according to the present invention that is derived from a particular HCV type 1b isolate. A "homologous HCV" would in this case be the same particular HCV type 1b isolate. "Homologous" in the context of "therapeutic treatment of a HCV homologous to the HCV peptides in a composition" has to be interpreted likewise.

With "prophylactic protection against infection by a heterologous HCV" is meant that protection is obtained against a challenge HCV virus classified in another genotype, subtype, or isolate as compared to the HCV genotype, subtype or isolate from which the HCV antigen or HCV antigens are derived. A composition may for example comprise a peptide or polypeptide according to the present invention that is derived from a particular HCV type 1b isolate. A "heterologous HCV" would in this case be, e.g., a HCV type 1b isolate sufficiently different from the type 1b isolate from which the antigens were derived, a type 1a HCV virus or a type 7 HCV virus. "Sufficiently different" as used in this particular context is to be understood at least a difference of 2%, 3% or 4% on the amino acid level. "Heterologous" in the context of "therapeutic treatment of a HCV heterologous to the HCV peptides in a composition" has to be interpreted likewise.

With the term "liver disease" is meant in this context any abnormal liver condition caused by infection with the hepatitis C virus including steatosis, inflammation, fibrosis, cirrhosis, necrosis, necro-inflammation and hepatocellular carcinoma.

With "reducing liver disease" is meant any stabilization or reduction of the liver disease status. Liver disease can be determined, e.g., by the Knodell scoring system (Knodell et al. 1981) or the Knodell scoring system adapted by Ishak (Ishak et al. 1995). A reduction of this score by two points is accepted as therapeutically beneficial effect in several studies (see, e.g., studies published after 1996 as indicated in Table 2 of Shiffman 1999).

With "reducing liver fibrosis progression" is meant any slowing down, halting or reverting of the normally expected progression of liver fibrosis. Liver fibrosis progression can be determined, e.g., by the Metavir scoring system. Normal expected progression of liver fibrosis according to this system was published to be an increase of the Metavir score of an untreated chronic HCV patient of approximately 0.133 per year (Poynard et al. 1997). "Reducing liver fibrosis" is meant to comprise any reduction of the normally expected progression of liver fibrosis.

Liver fibrosis and inflammation can be scored according to the Ishak scoring system (which is a modification of the scoring system of Knodell et al. 1981; Ishak et al. 1995) or Metavir scoring system (Bedossa and Poynard 1996). The Ishak scores range from 0 to 18 for grading of inflammation and from 0 to 6 for staging of fibrosis/cirrhosis. The sum of the Ishak inflammation and fibrosis scores comes closest to the Histological Activity Index (HAI; Knodell et al. 1981) which has been widely used. The Metavir scores range from 0 to 3 for grading of inflammation and from 0 to 4 for staging of fibrosis/cirrhosis. The overall progression rate of the Metavir score in an untreated patient is estimated to be 0.133 per year (Poynard et al. 1997).

Currently known HCV types include HCV genotypes 1, 2, 3, 4, 5, 6, 7, 8, 9, 10, 11 and known subtypes thereof include HCV subtypes 1a, 1b, 1c, 1d, 1e, 1f, 1g, 2a, 2b, 2c, 2d, 2e, 2f, 2g, 2h, 2i, 2k, 21, 3a, 3b, 3c, 3d, 3e, 3f, 3g, 4a, 4b, 4c, 4d, 4e, 4f, 4g, 4h, 4i, 4j, 4k, 41, 4m, 5a, 6a, 6b, 7a, 7b, 7c, 7d, 8a, 8b, 8c, 8d, 9a, 9b, 9c, 10a and 11a. The sequences of cDNA clones covering the complete genome of several prototype isolates have been determined and include complete prototype genomes of the HCV genotypes 1a (e.g., GenBank accession number AF009606), 1b (e.g., GenBank accession number AB016785), 1c (e.g., GenBank accession number D14853), 2a (e.g., GenBank accession number AB047639), 2b (e.g., GenBank accession number AB030907), 2c (e.g., GenBank accession number D50409) 2k (e.g., GenBank accession number AB031663), 3a (e.g., GenBank accession number AF046866), 3b (e.g., GenBank accession number D49374), 4a (e.g., GenBank accession number Y11604), 5a (e.g., GenBank accession number AF064490), 6a (e.g., GenBank accession number Y12083), 6b (e.g., GenBank accession number D84262), 7b (e.g., GenBank accession number D84263), 8b (e.g., GenBank accession number D84264), 9a (e.g., GenBank accession number D84265), 10a (e.g., GenBank accession number D63821) and 11a (e.g., GenBank accession number D63822). A new HCV genotype was further described in International Patent Publication No. WO03/020970. An HCV isolate is to be considered as a HCV quasispecies isolated from a HCV-infected mammal. A HCV quasispecies usually comprises a number of variant viruses with variant genomes usually of the same HCV type or HCV subtype.

A "pharmaceutically acceptable carrier" or "pharmaceutically acceptable adjuvant" is any suitable excipient, diluent, carrier and/or adjuvant which, by themselves, do not induce the production of antibodies harmful to the individual receiving the composition nor do they elicit protection. Preferably, a pharmaceutically acceptable carrier or adjuvant enhances the immune response elicited by an antigen. Suitable carriers or adjuvantia typically comprise one or more of the compounds included in the following non-exhaustive list:

- large slowly metabolized macromolecules such as proteins, polysaccharides, polylactic acids, polyglycolic acids, polymeric amino acids, amino acid copolymers and inactive virus particles;
- aluminium hydroxide, aluminium phosphate (see International Patent Application Publication No. WO93/24148), alum ($KAl(SO_4)_2 . 12H_2O$), or one of these in combination with 3-0-deacylated monophosphoryl lipid A (see International Patent Application Publication No. WO93/19780);
- N-acetyl-muramyl-L-threonyl-D-isoglutamine (see U.S. Patent No. 4,606,918), N-acetyl-normuramyl-L-alanyl-D-isoglutamine, N-acetylmuramyl-L-alanyl-D-isoglutamyl-L-alanine2-(1',2'-dipalmitoyl-sn-glycero-3-hydroxyphosphoryloxy) ethylamine;
- RIBI (ImmunoChem Research Inc., Hamilton, MT, USA) which contains monophosphoryl lipid A (i.e., a detoxified endotoxin), trehalose-6,6-dimycolate, and cell wall skeleton (MPL + TDM + CWS) in a 2% squalene/Tween 80 emulsion. Any of the three components MPL, TDM or CWS may also be used alone or combined 2 by 2. The MPL may also be replaced by its synthetic analogue referred to as RC-529 or by any other amino-alkyl glucosaminide 4-phosphate (Johnson et al. 1999, Persing et al. 2002);
- adjuvants such as Stimulon (Cambridge Bioscience, Worcester, MA, USA), SAF-1 (Syntex);
- bacterial DNA-based adjuvants such as ISS (Dynavax) or CpG (Coley Pharmaceuticals);
- adjuvants such as combinations between QS21 and 3-de-O-acetylated monophosphoryl lipid A (see International Patent Application Publication No. WO94/00153) which may be further supplemented with an oil-in-water emulsion (see, e.g., International Patent Application Publication Nos. WO95/17210, WO97/01640 and WO9856414) in which the oil-in-water emulsion comprises a metabolisable oil and a saponin, or a metabolisable oil, a saponin, and a sterol, or which may be further supplemented with a cytokine (see International Patent Application Publication No. WO98/57659);

- adjuvants such as MF-59 (Chiron), or poly[di(carboxylatophenoxy) phosphazene] based adjuvants (Virus Research Institute);

- blockcopolymer based adjuvants such as Optivax (Vaxcel, Cytrx) or inulin-based adjuvants, such as Algammulin and Gammalnulin (Anutech);

- Complete or Incomplete Freund's Adjuvant (CFA or IFA, respectively) or Gerbu preparations (Gerbu Biotechnik). It is to be understood that Complete Freund's Adjuvant (CFA) may be used for non-human applications and research purposes as well;

- a saponin such as QuilA, a purified saponin such as QS21, QS7 or QS17, p-escin or digitonin;

- immunostimulatory oligonucleotides comprising unmethylated CpG dinucleotides such as [purine-purine-CG-pyrimidine-pyrimidine] oligonucleotides. Immunostimulatory oligonucleotides may also be combined with cationic peptides as described, e.g., by Riedl et al. (2002);

- Immune Stimulating Complexes together with saponins, for example Quil A (ISCOMS);

- excipients and diluents, which are inherently non-toxic and non-therapeutic, such as water, saline, glycerol, ethanol, wetting or emulsifying agents, pH buffering substances, preservatives, and the like;

- a biodegradable and/or biocompatible oil such as squalane, squalene, eicosane, tetratetracontane, glycerol, peanut oil, vegetable oil, in a concentration of, e.g., 1 to 10% or 2.5 to 5%;

- vitamins such as vitamin C (ascorbic acid or its salts or esters), vitamin E (tocopherol), or vitamin A;

- carotenoids, or natural or synthetic flavanoids;

- trace elements, such as selenium;

- any Toll-like receptor ligand as reviewed in Barton and Medzhitov (2002).

Any of the afore-mentioned adjuvants comprising 3-de-O-acetylated monophosphoryl lipid A, said 3-de-O-acetylated monophosphoryl lipid A may be forming a small particle (see International Patent Application Publication No. WO94/21292).

A "pharmaceutically acceptable vehicle" includes vehicles such as water, saline, physiological salt solutions, glycerol, ethanol, etc. Auxiliary substances such as wetting or emulsifying agents, pH buffering substances, preservatives may be included in such vehicles.

Typically, a vaccine composition is prepared as an injectable, either as a liquid solution or suspension. Injection may be subcutaneous, intramuscular, intravenous, intraperitoneal, intrathecal, intradermal, intraepidermal. Other types of administration comprise implantation, suppositories, oral ingestion, enteric application, inhalation, aerosolization or nasal spray or drops. Solid forms, suitable for dissolving in, or suspension in, liquid vehicles prior to injection may also be prepared. The preparation may also be emulsified or encapsulated in liposomes for enhancing adjuvant effect.

Other aspects of the invention relate to methods of vaccinating a HCV-naive or HCV-infected mammal comprising administering an HCV immunogenic composition, an HCV vaccine composition, a prophylactic HCV vaccine composition and/or a therapeutic HCV vaccine composition according to the invention in combination with (i.e., before, after or concurrently with) administering a DNA vaccine.

The immunogenic composition, vaccine composition, therapeutic vaccine composition or prophylactic vaccine composition as described above may in addition comprise DNA vaccine vectors capable of expressing or effectuating expression of an antigen. Particularly relating to the current invention, the HCV immunogenic composition, HCV vaccine composition, therapeutic HCV vaccine composition or prophylactic HCV vaccine composition may in addition comprise DNA vaccine vectors capable of expressing or effectuating expression of one or more antigens such as HCV proteins or parts thereof, e.g., a HCV NS3 protein or part thereof. Alternatively, the protein- or peptide-based immunogenic composition, vaccine composition, therapeutic vaccine composition or prophylactic vaccine composition of the invention may be used in combination with a DNA vector-based immunogenic composition, vaccine composition, therapeutic vaccine composition or prophylactic vaccine composition (also referred to as "DNA vaccine" or "HCV DNA vaccine" if the DNA vector comprised therein is encoding a HCV protein or part thereof). Such combination for instance includes a DNA-prime protein-boost vaccination scheme wherein vaccination is initiated by administering a DNA vector-based immunogenic composition, vaccine composition, therapeutic vaccine composition or prophylactic vaccine composition and is followed by administering a protein- or peptide-based immunogenic composition, vaccine composition, therapeutic vaccine composition or prophylactic vaccine composition of the invention. In particular the DNA vaccine vector is capable of expressing one or more HCV antigens or proteins or parts thereof.

With a "DNA vector" or "DNA vaccine vector" is meant any DNA carrier comprising the open reading frame for one or more of the peptides useful for eliciting and/or enhancing an immune response. In general, said open reading frames are operably linked to transcription regulatory elements, such as promoters and terminators, enabling expression of the peptide encoded by the open reading frame. The terms "DNA vector" or "DNA vaccine vector" are meant to include naked plasmid DNA, plasmid DNA formulated with a suitable pharmaceutically acceptable carrier, recombinant viruses (e.g., as described above), or recombinant viruses formulated with a suitable pharmaceutically acceptable carrier. A "HCV DNA vector" or "HCV DNA vaccine vector" relates to any DNA carrier comprising the open reading frame for one or more of the HCV peptides.

As used herein, the term "transcription regulatory elements" refers to a nucleotide sequence which contains essential regulatory elements, such that upon introduction into a living vertebrate cell it is able to direct the cellular machinery to produce translation products encoded by the polynucleotide.

The term "operably linked" refers to a juxtaposition wherein the components are configured so as to perform their usual function. Thus, transcription regulatory elements operably linked to a nucleotide sequence are capable of effecting the expression of said nucleotide sequence. Those skilled in the art can appreciate that different transcriptional promoters, terminators, carrier vectors or specific gene sequences may be used successfully

Another aspect of the current invention relates to the use of an isolated protein or part thereof or derivative of any thereof according to the invention in immunoassays, to the incorporation of an isolated protein or part thereof or derivative of any thereof according to the invention in immunoassay kits or diagnostic kits, and to the use of an isolated protein or part thereof or derivative of any thereof according to the invention for the manufacture of a an immunoassay kit or diagnostic kit. Immunoassays comprise immunological methods for determining the presence of antibodies to HCV in a biological sample or of antigens of HCV in a biological sample or of HCV virus in a biological sample, or for diagnosing HCV infection. Diagnostic kits or immunoassay kits comprise kits for determining the presence of antibodies to HCV in a biological sample or of antigens of HCV in a biological sample or of HCV virus in a biological sample, or for diagnosing HCV infection.

In particular said biological sample is suspected to contain HCV antibodies, HCV antigens or HCV virus.

A first general embodiment in relation to immunoassays comprises a method for determining the presence of antibodies to HCV, in particular to HCV NS3, in a biological sample comprising the step of detecting said antibodies to an isolated protein or part thereof or derivative of any thereof according to the invention.

A second general embodiment in relation to immunoassays comprises a method for determining the presence of HCV NS3 antigens in a biological sample comprising the step of detecting said HCV NS3 antigens with an antibody to said HCV NS3 antigens in the presence of an isolated protein or part thereof or derivative of any thereof according to the invention as competitor of binding of said HCV NS3 antigens to said antibody.

A first specific embodiment in relation to immunoassays comprises a method for determining the presence of antibodies to HCV, in particular to HCV NS3, in a biological sample comprising the steps of:

(i) contacting said biological sample with an isolated protein or part thereof or derivative of any thereof according to the invention;
(ii) detecting the immunological complex formed between said antibodies and said protein or part thereof or derivative of any thereof.

A second specific embodiment in relation to immunoassays comprises a method for determining the presence of a HCV virus in a biological sample comprising the steps of:

(i) contacting said biological sample with an isolated protein or part thereof or derivative of any thereof according to the invention;
(ii) detecting the immunological complex formed between antibodies to said HCV virus present in said sample and said protein or part thereof or derivative of any thereof;
(iii) inferring from the immunological complex formed in (ii) the presence of a HCV virus in said biological sample.

A third specific embodiment in relation to immunoassays comprises a method for diagnosing HCV infection in a mammal comprising the steps of:

(i) contacting a biological sample from said mammal with an isolated protein or part thereof or derivative of any thereof according to the invention;
(ii) detecting the immunological complex formed between antibodies to HCV present in said sample and said protein or part thereof or derivative of any thereof;
(iii) diagnosing from the immunological complex formed in (ii) HCV infection in said mammal.

A forth specific embodiment in relation to immunoassays comprises a method for determining the presence of a HCV NS3 antigen in a biological sample comprising the steps of:

(i) contacting said biological sample with an antibody to said HCV NS3 antigen in the presence of an isolated protein or part thereof or derivative of any thereof according to the invention as competitor, i.e. as competitor of binding of said HCV NS3 antigen to said antibody;
(ii) inferring from the immunological complex formed between said antibodies and said HCV NS3 antigen the presence of said HCV NS3 antigen.

A fifth specific embodiment in relation to immunoassays comprises a method for determining the presence of a HCV virus in a biological sample comprising the steps of:

(i) contacting said biological sample with an antibody to an HCV NS3 antigen in the presence of an isolated protein or part thereof or derivative of any thereof according to the invention as competitor;
(ii) detecting the immunological complex formed between said antibodies and said HCV NS3 antigen;
(iii) inferring from the immunological complex formed in (ii) the presence of a HCV virus in said biological sample.

A sixth specific embodiment in relation to immunoassays comprises a method for diagnosing HCV infection in a mammal comprising the steps of:

(i) contacting a biological sample from said mammal with an antibody to an HCV NS3 antigen in the presence of an isolated protein or part thereof or derivative of any thereof according to the invention as competitor;
(ii) detecting the immunological complex formed between said antibodies and said HCV NS3 antigen;
(iii) diagnosing from the immunological complex formed in (ii) HCV infection in said mammal.

A further embodiment relates to the use of a protein or part thereof or derivative of any thereof according to the invention in an immunoassay.
Yet another embodiment relates to the use of a protein or part thereof or derivative of any thereof according to the invention in the manufacture of an immunoassay or immunoassay kit.
A further embodiment relates to a diagnostic kit for determining the presence of antibodies to HCV (in particular to HCV NS3) in a biological sample, for determining the presence of HCV NS3 antigens in a biological sample, for determining the presence of a HCV virus in a biological sample or for diagnosing HCV infection in a mammal, said kit comprising an isolated protein or part thereof or derivative of any thereof according to the invention.
The protein or part thereof or derivative of any thereof according to the present invention may be employed in virtually any immunoassay format that employs a known antigen to detect antibodies or a known antibody to detect antigens. A common feature of all of these assays is that the antigen is contacted with the body component containing or suspected of containing HCV antibodies or HCV antigens under conditions that permit binding between an antigen and an antibody, i.e. under conditions allowing the formation of an immunological complex. Such conditions will typically be physiologic temperature, pH and ionic strength using an excess of antigen (in the case of antibody detection) or antibody (in the case of antigen detection). The incubation of the antigen or antibody with the specimen is followed by detection of immune complexes.
The design of immunoassays is subject to a great deal of variation, and many formats are known in the art. Protocols may, for example, use solid supports, or immunoprecipitation. Most assays involve the use of labeled antibody and/or labeled polypeptide, e.g. a labeled peptide or polypeptide according to the present invention; the labels may be, for example, enzymatic, fluorescent, chemiluminescent, radioactive, or dye molecules. Assays which amplify the signals from the immune complex are also known; examples of which are assays which utilize biotin and avidin or streptavidin, and enzyme-labeled and mediated immunoassays, such as ELISA and RIA assays. Other immunoassay designs comprise line immunoassays, sandwich immunoassays, antigen down immunoassays. An immunoassays may be set up in a competitive format.
An immunoassay may be, without limitation, in a heterogeneous or in a homogeneous format, and of a standard or competitive type. In a heterogeneous format, the polypeptide is typically bound to a solid matrix, solid support or solid phase to facilitate separation of the sample from the polypeptide after incubation. Examples of solid supports, matrices or phases are listed furtheron. The solid support containing the antigenic polypeptides is typically washed after separating it from the test sample, and prior to detection of bound antibodies. Both standard and competitive formats are know in the art.
In a homogeneous format, the test sample is incubated with the combination of antigens in solution. For example, it may be under conditions that will precipitate any antigen-antibody complexes which are formed. Both standard and competitive formats for these assays are known in the art.
In a standard format, the amount of antibodies, such as anti-HCV antibodies, in the antibody-antigen complexes is directly monitored. This may be accomplished by determining whether labeled anti-xenogeneic (e.g. anti-human) antibodies which recognize an epitope on said antibodies, such as said anti-HCV antibodies, will bind due to complex formation. In a competitive format, the amount of said antibodies, such as said anti-HCV antibodies, in a sample is deduced by monitoring the competitive effect on the binding of a known amount of (labeled) antibody (or other competing ligand) or antigen in the complex.

Antigen-antibody complexes can be detected by any of a number of known techniques, depending on the format. For example, unlabeled antibodies such as anti-HCV antibodies in the complex may be detected using a conjugate of anti-xenogeneic 1g complexed with a label (e.g. an enzyme label).

In an immunoprecipitation or agglutination assay format the reaction between an antigen and an antibody forms a protein cluster that precipitates from the solution or suspension and forms a visible layer or film of precipitate. If no antibody is present in the test specimen or sample, no such precipitate is formed.

A diagnostic kit usually comprises a molecule for detecting the presence of a sample reactant capable of interacting with said molecule, of a sample reactant modifying said molecule (e.g., in a chemical reaction), and/or of a sample reactant modifiable by said molecule (e.g., in a chemical reaction). In a diagnostic kit for detection of an antigen or antibody in a sample, one or more antibodies or antigens, respectively, are part of said kit. In a diagnostic kit for detecting antigens or antibodies, antibodies or antigens, respectively, are often present on a solid phase, matrix or support.

The proteins or parts thereof or derivatives of any thereof according to the present invention can be packaged and be part of a diagnostic kit. The kit will normally contain in separate containers or vials the peptides or polypeptides according to the present invention (labelled or unlabelled), control antibody formulations (positive and/or negative), labelled antibody when the assay format requires the same and signal generating reagents (e.g. enzyme substrate) if the label does not generate a signal directly. The peptides or polypeptides according to the present invention may be already bound to a solid matrix or may be present in the kit in a separate vial together with reagents for binding it to the matrix. Instructions (e.g. written, tape, CD-ROM, etc.) for carrying out the assay usually will be included in the kit.

The signal generating compound can include an enzyme, a luminescent compound, a chromogen, a radioactive element and a chemiluminescent compound. Examples of enzymes include alkaline phosphatase, horseradish peroxidase and beta-galactosidase. Examples of enhancer compounds include biotin, anti-biotin and avidin. Examples of enhancer compounds binding members include biotin, anti-biotin and avidin. In order to block the effects of rheumatoid factor-like substances, the test sample is subjected to conditions sufficient to block the effect of rheumatoid factor-like substances. These conditions comprise contacting the test sample with a quantity of anti-human IgG to form a mixture, and incubating the mixture for a time and under conditions sufficient to form a reaction mixture product substantially free of rheumatoid factor-like substance.

Solid phases, solid matrices or solid supports on which molecules, e.g., the antigens of the present invention, may be bound (or captured, absorbed, adsorbed, linked, coated, immobilized; covalently or non-covalently) comprise beads or the wells or cups of microtiter plates, or may be in other forms, such as solid or hollow rods or pipettes, particles, e.g., from 0.1 $\mu$m to 5 mm in diameter (e.g. "latex" particles, protein particles, or any other synthetic or natural particulate material), microspheres or beads (e.g. protein A beads, magnetic beads). A solid phase may be of a plastic or polymeric material such as nitrocellulose, polyvinyl chloride, polystyrene, polyamide, polyvinylidine fluoride or other synthetic polymers. Other solid phases include membranes, sheets, strips, films and coatings of any porous, fibrous or bibulous material such as nylon, polyvinyl chloride or another synthetic polymer, a natural polymer (or a derivative thereof) such as cellulose (or a derivative thereof such as cellulose acetate or nitrocellulose). Fibers or slides of glass, fused silica or quartz are other examples of solid supports. Paper, e.g., diazotized paper may also be applied as solid phase. Clearly, molecules, *in casu* the antigens of the present invention, may be bound, captured, absorbed, adsorbed, linked or coated to any solid phase suitable for use in immunoassays. Said molecules, *in casu* the antigens of the present invention, may be present on a solid phase in defined zones such as spots or lines.

Any of the above described solid phases may be developed, e.g. automatically developed in an assay device.

With "developed" or "development" is meant that a sample or samples, suspected of comprising a binding partner to a molecule present on a solid phase, is or are applied to said solid phase and that the necessary steps are performed in order to detect binding of the binding partner to a molecule on a solid phase. This can, e.g., be the detection of binding of an antibody suspected to be present in a biological sample to an antigen, *in casu* an antigen of the present invention, present on a solid phase. Automatic development hence refers to a development process, or any one or more steps thereof, in an automated or robotized fashion.

A development automate or robot (or, generally, an assay device) generally is connected to or comprises one, more or all of the development or assay reagents and may in addition comprise a means to "read" the developed assay. Said "reading" will logically depend on the assay and may, e.g., confer to determining color intensities, to determining optical density or absorption at a given wavelength, to determining fluoresence, fosforescence or (chemi)luminescence, to determining turbidity, to determining the decay of a radio-active element or to determining other physical or physicochemical characteristics that are related to the binding of a binding partner in a sample to a molecule present on a solid phase.

A biological sample may be a liquid test sample or a solid test sample. A liquid test sample may be any body fluid, for example, blood, plasma, serum, saliva, urine, cerebro-spinal fluid, milk, lymph fluid, tears, or secretions of the respiratory, intestinal or genito-urinary tracts. A solid test sample such as cells or tissue may be brought into liquid form for testing, for example, as tissue exudate or macerate. A solid test sample such as cells or tissue may be fixed, or fixed and sectioned, an example thereof being formalin-fixed paraffin-embedded liver tissue sections.

## EXAMPLES

### EXAMPLE 1. Production of NS3 in *Escherichia coli.*

As an example of HCV NS3 protein production, production of the HCV NS3-TN protein is herein given. This production method can, however, be applied to other HCV NS3 proteins (or fragments thereof) as well. The HCV NS3-TN protein (amino acids 1166-1468 of the HCV polyprotein in which the amino acids 1167 to 1180 have been replaced by the amino acids 1071-1084, as described in Example 7a of International Patent Application No. PCT/EP99/04342 (Publication No. WO 99/67285)) was expressed in *E. coli.*

The NS3-TN protein (SEQ ID NO:1) was purified essentially as described in Example 7b of International Patent Application No. PCT/EP99/04342 (Publication No. WO 99/67285) making use of sulfonation as modifying agent for the cysteines, thus yielding sulfonated NS3-TN (NS3-TN SO3).

Alternatively, cysteine thiol-groups in the NS3-TN protein were blocked by means of alkylation with iodoacetamide. Thereto, NS3-TN SO3 was incubated in 50 mM DTT for 30 minutes at 37°C followed by an alkylation step in which iodoacetamide was added to a final concentration of 200 mM (30 minutes at 37°C). This yielded the alkylated NS3-TN (NS3-TN IAA).

Finally the NS3-TN SO3 and NS3-TN IAA material was desalted to PBS, pH 7.5 containing 6 M urea. NS3-TN SO3 was thus obtained at 1.45 mg/mL, and NS3-TN IAA at 1.9 mg/mL.

The natural contiguous NS3 amino acid sequence (i.e. amino acids 1181-1468) are defined in SEQ ID NO: 2. The amino acids 1071-1084 are defined in SEQ ID NO:3. The amino acids 1073-1084 are defined in SEQ ID NO:4.

### EXAMPLE 2. Antigenicity study of NS3 in ELISA.

The sulfonated and alkylated NS3 batches from Example 1 were compared by ELISA with serum samples derived from HCV carriers or healthy donors. The sulfonated NS3 was analyzed as such but also after desulfonation. Coating was at 3 µg/ml in PBS, and for desulfonation 5 mM DTT was added to the coating buffer. The results are shown in Table 1. Based on the average reactivity shown at the bottom of the table in gray shading, both the alkylated and sulfonated (with or without DTT) have a very low reactivity with sera from healthy donors. There is however, a clear need for the sulfonated protein to be treated with DTT to improve the response with serum from HCV carriers. In the case of serum 17805 this sample would have been wrongly interpreted as negative for HCV antibodies if the sulfon groups would not have been removed. Surprisingly the alkyl groups interfere far less with detection of antibodies and the average reactivity of this protein is very similar to the reactivity of the desulfonated protein.

### EXAMPLE 3. Immunogenicity of NS3 in mice.

The purified NS3-TN SO3 and NS3-TN IAA proteins obtained as described in Example 1 were diluted to 500 µg/mL with 0.9% NaCl, mixed with an equal volume of Alhydrogel 1.3% (Superfos, Denmark) and finally further diluted with 8 volumes of 0.9% NaCl to yield alum-adjuvanted NS3 at a concentration of 50 µg NS3-TN/mL and 0.13% of Alhydrogel.

Groups of 6 Balb/c mice were immunized intramuscularly three times, with a three-week interval, with 5 µg of either sulfonated or alkylated NS3. The immune response was assessed 2 weeks after the third immunization.

### ANTIBODY TITERS

Antibody titers were determined by ELISA. After coating with the specific (desulfonated with 5mM DTT for 1hr at 37°C) antigen form (3 µg/mL, overnight at 4°C) and blocking, serum was incubated in sample diluent. Highest (starting) serum concentration was 1/1000, and this concentration was further diluted, each time with 0.5 log 10 (or 1/3.16). As a conjugate, HRP labelled rabbit anti-mouse 1g (1/20 000, stock concentration of 1.3 mg/mL, DAKO) was used. For each titration, the log EC 50 values were calculated by Prism using following parameters: non-linear regression, sigmoidal dose-response curve with fixed bottom value (= blanc). The results are summarized in Figure 1.

All animals mounted an antibody response against NS3. Both the alkylated and the sulfonated protein induce a significant antibody response which can be detected both by alkylated or desulfonated protein in the ELISA. From Figure 2, it can be judged that the sulfonated protein induces higher antibody levels; This observation is irrespective of the protein used to determine the antibody titers in ELISA.

T-CELL IMMUNITY

After isolation and counting, mice spleen cells were plated out at a concentration of 200 000 cells per well in flat bottom 96 well plates and were restimulated in vitro with medium alone or with the different antigens at a final concentration of 1 µg/mL. After 5 days of culturing, $^3$H-thymidine (1 µCi/well) was incorporated overnight and cells were harvested the next morning. All experiments were performed in five fold. The results in figure are expressed as stimulation index (SI) . The SI is calculated with the following formula:

$$\frac{\text{mean cpm of 5 cultures stimulated with antigen}}{\text{mean cmp of 5 cultures stimulated without antigen}}$$

The mean SI, as can be judged from the Figure 2 tends to be higher for immunization with alkylated NS3 and this response can be restimulated similarly well with alkylated or sulfonated protein. The sulfonated NS3 seems to induce by immunization somewhat lower T-cell responses which can hardly not be restimulated in vitro by alkylated proteins. In conclusion, both the sulfonated and alkylated NS3 induce high T-cell responses in vivo as judged by the in vitro restimulation by sulfonated NS3 which we assume is mimicking best the natural NS3 as the sulfon groups can be removed by the antigen presenting cells in this assay. There, is however a qualitative and potentially also a quantitative difference in this immune response especially when considering the restimulation in vitro by alkylated protein.

**Table 1:** OD values as obtained in ELISA with sera from HCV carriers or healthy donors. The sera were incubated at a dilution of 1/20 on the NS3 coated plates. Ser nr = serum number. Avg = average.

| HCV-sera Ser nr | NS3 IAA | NS3 SO3 | NS3 SO3 +DTT | Healthy donor sera Ser nr | NS3 IAA | NS3 SO3 | NS3 SO3 +DTT |
|---|---|---|---|---|---|---|---|
| 17807 | 1.518 | 1.011 | 1.595 | F504 | 0.068 | 0.064 | 0.051 |
| 17842 | 1.522 | 0.292 | 1.569 | F511 | 0.062 | 0.063 | 0.050 |
| 17777 | 1.588 | 1.416 | 1.547 | F516 | 0.058 | 0.061 | 0.048 |
| 17785 | 1.579 | 1.421 | 1.550 | F517 | 0.061 | 0.058 | 0.053 |
| 17794 | 1.444 | 1.220 | 1.396 | F518 | 0.070 | 0.095 | 0.091 |
| 17798 | 1.149 | 0.944 | 1.433 | F519 | 0.143 | 0.134 | 0.110 |
| 17805 | 1.101 | 0.118 | 1.525 | F520 | 0.128 | 0.142 | 0.073 |
| 17810 | 1.698 | 1.267 | 1.706 | F521 | 0.114 | 0.143 | 0.088 |
| 17819 | 1.756 | 1.472 | 1.582 | F522 | 0.071 | 0.073 | 0.062 |
| 17826 | 1.574 | 1.208 | 1.544 | F523 | 0.095 | 0.189 | 0.125 |
| 17849 | 1.578 | 1.408 | 1.773 | F526 | 0.087 | 0.084 | 0.054 |
| 17763 | 1.596 | 1.427 | 1.701 | F529 | 0.086 | 0.085 | 0.079 |
| 17807 | 1.518 | 0.972 | 1.660 | F513 | 0.120 | 0.135 | 0.100 |
| 17808 | 1.455 | 1.156 | 1.639 | F530 | 0.064 | 0.067 | 0.050 |
| 17816 | 1.441 | 0.994 | 1.538 | F531 | 0.094 | 0.100 | 0.049 |
| 17820 | 0.346 | 0.304 | 1.013 | F527 | 0.098 | 0.092 | 0.074 |
| 55333 | 1.644 | 1.464 | 1.607 | F532 | 0.104 | 0.087 | 0.049 |
| 55337 | 1.625 | 1.211 | 1.403 | F533 | 0.089 | 0.108 | 0.079 |
| 55340 | 1.687 | 1.163 | 1.621 | F534 | 0.072 | 0.060 | 0.050 |
| 55342 | 1.723 | 1.251 | 1.561 | F535 | 0.067 | 0.077 | 0.055 |
| 55345 | 1.679 | 1.469 | 1.689 | F536 | 0.088 | 0.091 | 0.054 |
| 55348 | 1.436 | 0.987 | 1.526 | F552 | 0.072 | 0.073 | 0.065 |
| 55350 | 1.649 | 0.907 | 1.614 | F553 | 0.071 | 0.056 | 0.050 |
| 55352 | 1.341 | 0.905 | 1.427 | F555 | 0.066 | 0.063 | 0.044 |
| 55353 | 1.294 | 0.843 | 1.332 | avg | 0.085 | 0.092 | 0.067 |
| 55354 | 0.770 | 0.597 | 0.902 | | | | |
| 55355 | 1.306 | 0.911 | 1.315 | | | | |
| 55362 | 1.222 | 0.937 | 1.498 | | | | |
| 55365 | 1.396 | 1.365 | 1.364 | | | | |
| avg | 1.436 | 1.057 | 1.504 | | | | |

**REFERENCES**

1. Barton,G.M. & Medzhitov,R. Toll-like receptors and their ligands. *Curr. Top. Microbiol. Immunol.* **270,** 81-92 (2002).

2. Bedossa,P. & Poynard,T. An algorithm for the grading of activity in chronic hepatitis C. The METAVIR Cooperative Study Group. *Hepatology* **24**, 289-293 (1996).

3. Beekman,N.J. *et al.* Synthetic peptide vaccines: palmitoylation of peptide antigens by a thioester bond increases immunogenicity. *J. Pept. Res.* **50,** 357-364 (1997).

4. Bums,J., Butler,J. & Whitesides,G. Selective reduction of disulfides by Tris(2-carboxyethyl)phosphine. *J. Org. Chem.* **56,** 2648-2650 (1991).

5. Choo,Q.L. *et al.* Vaccination of chimpanzees against infection by the hepatitis C virus. *Proc. Natl. Acad. Sci. U. S. A* **91**, 1294-1298 (1994).

6. Darbre,A. Practical protein chemistry: a handbook. Whiley & Sons Ltd., (1986).

7. Foms,X. *et al.* DNA immunization of mice and macaques with plasmids encoding hepatitis C virus envelope E2 protein expressed intracellularly and on the cell surface. *Vaccine* **17,** 1992-2002 (1999).

8. Foms,X. *et al.* Vaccination of chimpanzees with plasmid DNA encoding the hepatitis C virus (HCV) envelope E2 protein modified the infection after challenge with homologous monoclonal HCV. *Hepatology* **32**, 618-625 (2000).

9. Fujihashi,K. *et al.* Cytokine-specific ELISPOT assay. Single cell analysis of IL-2, IL-4 and IL-6 producing cells. *J. Immunol. Methods* **160,** 181-189 (1993).

10. Gailit,J. Restoring free sulfhydryl groups in synthetic peptides. *Anal. Biochem.* **214**, 334-335 (1993).

11. Grakoui,A., Wychowski,C., Lin,C., Feinstone,S.M. & Rice,C.M. Expression and identification of hepatitis C virus polyprotein cleavage products. *J. Virol.* **67,** 1385-1395 (1993).

12. Hermanson,G.T. Bioconjugate techniques. Academic Press, San Diego (1996).

13. Holmgren,A. Thioredoxin catalyzes the reduction of insulin disulfides by dithiothreitol and dihydrolipoamide. *J. Biol. Chem.* **254**, 9627-9632 (1979).

14. Houghton,M. *et al.* Prospects for prophylactic and therapeutic hepatitis C virus vaccines. *Princess Takamatsu Symp.* **25**, 237-243 (1995).

15. Hu,G.J., Wang,R.Y., Han,D.S., Alter,H.J. & Shih,J.W. Characterization of the humoral and cellular immune responses against hepatitis C virus core induced by DNA-based immunization. *Vaccine* **17**, 3160-3170 (1999).

16. Ishak,K. *et al.* Histological grading and staging of chronic hepatitis. *J. Hepatol.* **22**, 696-699 (1995).

17. Johnson,D.A. *et al.* Synthesis and biological evaluation of a new class of vaccine adjuvants: aminoalkyl glucosaminide 4-phosphates (AGPs). *Bioorg. Med. Chem Lett* **9**, 2273-2278 (1999).

18. Knodell,R.G. *et al.* Formulation and application of a numerical scoring system for assessing histological activity in asymptomatic chronic active hepatitis. *Hepatology* **1**, 431-435 (1981).

19. Kumar,N., Kella,D. & Kinsella,J.E. A method for the controlled cleavage of disulfide bonds in proteins in the absence of denaturants. *J. Biochem. Biophys. Methods* **11**, 251-263 (1985).

20. Kumar,N., Kella,D. & Kinsella,J.E. Anomalous effects of denaturants on sulfitolysis of protein disulfide bonds. *Int. J. Peptide Prot. Res.* **28**, 586-592 (1986).

21. Lauer,G.M. & Walker,B.D. Hepatitis C virus infection. *N. Engl J. Med.* **345,** 41-52 (2001).

22. Lopez-Dias de Cerio AL *et al.* T(h)1 but not T(h)0 cell help is efficient to induce cytotoxic T lymphocytes by immunization with short synthetic peptides. *Int Immunol.* **11**, 2025-2034 (1999).

23. Mori,S., Ohkoshi,S., Hijikata,M., Kato,N. & Shimotohno,K. Serodiagnostic assay of hepatitis C virus infection using viral proteins expressed in Escherichia coli. *Jpn. J. Cancer Res* **83,** 264-268 (1992).

24. Persing,D. *et al.* Taking toll: lipid A mimetics as adjuvants and immunomodulators. *Trends Microbiol.* **10,** S32 (2002).

25. Pomroy,N.C. & Deber,C.M. Solubilization of hydrophobic peptides by reversible cysteine PEGylation. *Biochem. Biophys. Res. Commun.* **245,** 618-621 (1998).

26. Poynard,T., Bedossa,P. & Opolon,P. Natural history of liver fibrosis progression in patients with chronic hepatitis C. The OBSVIRC, METAVIR, CLINIVIR, and DOSVIRC groups. *Lancet* **349**, 825-832 (1997).

27. Rein,A. *et al.* Inactivation of murine leukemia virus by compounds that react with the zinc finger in the viral nucleocapsid protein. *J. Virol.* **70**, 4966-4972 (1996).

28. Riedl,P., Buschle,M., Reimann,J. & Schirmbeck,R. Binding immune-stimulating oligonucleotides to cationic peptides from viral core antigen enhances their potency as adjuvants. *Eur. J. Immunol.* **32,** 1709-1716 (2002).

29. Shiffman,M.L. Improvement in liver histopathology associated with interferon therapy in patients with chronic hepatitis C. *Viral Hepatitis Reviews* **5**, 27-43 (1999).

30. Shimotohno,K. *et al.* Processing of the hepatitis C virus precursor protein. *J. Hepatol.* **22,** 87-92 (1995).

31. Shirai,M. *et al.* Use of intrinsic and extrinsic helper epitopes for in vivo induction of anti-hepatitis C virus cytotoxic T lymphocytes (CTL) with CTL epitope peptide vaccines. *J. Infect. Dis.* **173,** 24-31 (1996).

32. Singh,R. & Kats,L. Catalysis of reduction of disulfide by selenol. *Anal. Biochem. 232,* 86-91 (1995).

33. Song,M.K., Lee,S.W., Suh,Y.S., Lee,K.J. & Sung,Y.C. Enhancement of immunoglobulin G2a and cytotoxic T-lymphocyte responses by a booster immunization with recombinant hepatitis C virus E2 protein in E2 DNA-primed mice. *J Virol.* **74,** 2920-2925 (2000).

34. Thakur,M.L., DeFulvio,J., Richard,M.D. & Park,C.H. Technetium-99m labeled monoclonal antibodies: evaluation of reducing agents. *Int. J. Rad. Appl. Instrum. B* **18**, 227-233 (1991).

35. Uno-Furuta,S. *et al.* Induction of virus-specific cytotoxic T lymphocytes by in vivo electric administration of peptides. *Vaccine* **19,** 2190-2196 (2001).

36. Vingerhoeds,M.H. *et al.* Immunoliposomes as enzyme-carriers (immuno-enzymosomes) for antibody-directed enzyme prodrug therapy (ADEPT): optimization of prodrug activating capacity. *Pharm. Res.* **13**, 604-610 (1996).

37. Walewski,J.L., Keller,T.R., Stump,D.D. & Branch,A.D. Evidence for a new hepatitis C virus antigen encoded in an overlapping reading frame. *RNA.* **7**, 710-721 (2001).

38. Xu,Z. *et al.* Synthesis of a novel hepatitis C virus protein by ribosomal frameshift. *EMBO J.* **20,** 3 840-3 848 (2001).

## Claims

1. An isolated HCV NS3 protein or part thereof wherein at least one cysteine thiol group is irreversibly modified for use as a medicament.

2. The isolated HCV NS3 protein or part thereof according to claim 1 wherein said medicament is an immunogenic composition or an HCV vaccine.

3. The isolated HCV NS3 protein or part thereof according to claim 1 or 2 wherein said HCV NS3 protein or part thereof comprises one or more amino acid derivatives, amino acid insertions, amino acid deletions or amino acid substitutions, or is comprised in a fusion protein.

4. The isolated HCV NS3 protein or part thereof according to any one of claims 1 to 3 wherein said at least one cysteine thiol group is modified by irreversible alkylation.

5. Use of an HCV NS3 protein or part thereof wherein at least one cysteine thiol group is irreversibly modified for the manufacture of a medicament for the prevention of infection of a mammal with HCV or for the treatment of an HCV-infected mammal.

6. The use according to claim 5 wherein said medicament is an immunogenic composition or an HCV vaccine.

7. The use according to claim 5 or 6 said HCV NS3 protein or part thereof comprises one or more amino acid derivatives, amino acid insertions, amino acid deletions or amino acid substitutions, or is comprised in a fusion protein.

8. The use according to any one of claims 5 to 7 wherein said at least one cysteine thiol group is modified by irreversible alkylation.

9. The use according to any one of claims 5 to 8 wherein said HCV immunogenic composition or HCV vaccine composition further comprises a DNA vector.

**FIGURE 1**

**FIGURE 2**

EP 1 481 985 A1

European Patent Office

**EUROPEAN SEARCH REPORT**

Application Number

EP 04 10 2409

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 99/67285 A (WIJNENDAELE FRANS VAN ;BOSMAN ALFONS (BE); DEPLA ERIK (BE); INNOGE) 29 December 1999 (1999-12-29)<br>* page 5, line 9 - page 5, line 14 *<br>* page 15, line 6 - page 15, line 30 *<br>* page 21, line 25 - page 21, line 31; claims; examples * | 1-9 | C07K14/18<br>A61K39/29<br>A61P31/14<br>G01N33/53<br>G01N33/576 |
| X | NARJES F ET AL: "A designed P1 cysteine mimetic for covalent and non-covalent inhibitors of HCV NS3 protease"<br>BIOORGANIC & MEDICINAL CHEMISTRY LETTERS, OXFORD, GB,<br>vol. 12, no. 4, 2002, pages 701-704,<br>XP002228005<br>ISSN: 0960-894X<br>* table 1 * | 1-9 | |
| | | | TECHNICAL FIELDS SEARCHED (Int.Cl.7)<br><br>C07K |

The present search report has been drawn up for all claims

| Place of search | Date of completion of the search | Examiner |
|---|---|---|
| Munich | 17 September 2004 | Meyer, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

28

## ANNEX TO THE EUROPEAN SEARCH REPORT
## ON EUROPEAN PATENT APPLICATION NO.

EP 04 10 2409

This annex lists the patent family members relating to the patent documents cited in the above-mentioned European search report.
The members are as contained in the European Patent Office EDP file on
The European Patent Office is in no way liable for these particulars which are merely given for the purpose of information.

17-09-2004

| Patent document cited in search report | | Publication date | Patent family member(s) | | Publication date |
|---|---|---|---|---|---|
| WO 9967285 | A | 29-12-1999 | AU | 765940 B2 | 02-10-2003 |
| | | | AU | 4615299 A | 10-01-2000 |
| | | | BR | 9911397 A | 15-01-2002 |
| | | | CA | 2330526 A1 | 29-12-1999 |
| | | | CN | 1313864 T | 19-09-2001 |
| | | | WO | 9967285 A1 | 29-12-1999 |
| | | | EP | 1090033 A1 | 11-04-2001 |
| | | | HU | 0102478 A2 | 28-10-2001 |
| | | | JP | 2002518037 T | 25-06-2002 |
| | | | NZ | 508797 A | 27-02-2004 |
| | | | PL | 345018 A1 | 19-11-2001 |
| | | | TR | 200003843 T2 | 21-06-2001 |
| | | | US | 6635257 B1 | 21-10-2003 |
| | | | US | 2003202987 A1 | 30-10-2003 |
| | | | US | 2003095980 A1 | 22-05-2003 |
| | | | US | 2003118603 A1 | 26-06-2003 |
| | | | ZA | 200007318 A | 10-03-2003 |

EPO FORM P0459

For more details about this annex : see Official Journal of the European Patent Office, No. 12/82

SEQUENCE LISTING

<110>   Innogenetics N.V.

<120>   Modified HCV NS3

<130>   152

<160>   4

<170>   PatentIn version 3.1

<210>   1
<211>   300
<212>   PRT
<213>   hepatitis C virus

<220>
<221>   MISC_FEATURE
<222>   (5)..(5)
<223>   Xaa may be Ile, Val or Thr

<220>
<221>   MISC_FEATURE
<222>   (8)..(8)
<223>   Xaa may be Val or Ala

<400>   1

Met Ala Thr Cys Xaa Asn Gly Xaa Cys Trp Thr Val Tyr His Gly Arg
1               5                   10                  15

Ala Ala Val Cys Thr Arg Gly Val Ala Lys Ala Val Asp Phe Val Pro
            20                  25                  30

Val Glu Ser Met Glu Thr Thr Met Arg Ser Pro Val Phe Thr Asp Asn
            35                  40                  45

```
Ser Ser Pro Pro Ala Val Pro Gln Thr Phe Gln Val Ala His Leu His
    50              55              60

Ala Pro Thr Gly Ser Gly Lys Ser Thr Lys Val Pro Ala Ala Tyr Ala
65              70              75              80

Ala Gln Gly Tyr Lys Val Leu Val Leu Asn Pro Ser Val Ala Ala Thr
            85              90              95

Leu Gly Phe Gly Ala Tyr Met Ser Lys Ala His Gly Val Asp Pro Asn
            100             105             110

Ile Arg Thr Gly Val Arg Thr Ile Thr Thr Gly Ala Pro Ile Thr Tyr
        115             120             125

Ser Thr Tyr Gly Lys Phe Leu Ala Asp Gly Gly Cys Ser Gly Gly Ala
    130             135             140

Tyr Asp Ile Ile Ile Cys Asp Glu Cys His Ser Ile Asp Ser Thr Ser
145             150             155             160

Ile Leu Gly Ile Gly Thr Val Leu Asp Gln Ala Glu Thr Ala Gly Ala
            165             170             175

Arg Leu Val Val Leu Ala Thr Ala Thr Pro Pro Gly Ser Val Thr Val
            180             185             190

Pro His Pro Asn Ile Glu Glu Val Ala Leu Ser Ser Thr Gly Glu Ile
            195             200             205

Pro Phe Tyr Gly Lys Ala Ile Pro Ile Glu Val Ile Lys Gly Gly Arg
    210             215             220

His Leu Ile Phe Cys His Ser Lys Lys Lys Cys Asp Glu Leu Ala Ala
225             230             235             240

Lys Leu Ser Gly Phe Gly Ile Asn Ala Val Ala Tyr Tyr Arg Gly Leu
            245             250             255

Asp Val Ser Val Ile Pro Thr Ser Gly Asp Val Val Val Val Ala Thr
            260             265             270

Asp Ala Leu Met Thr Gly Phe Thr Gly Asp Phe Asp Ser Val Ile Asp
            275             280             285

Cys Asn Thr Cys Val Thr Gln Thr Val Asp Phe Ser
    290             295             300
```

<210> 2

<211> 278

<212>   PRT

<213>   hepatitis C virus


<400>   2

Gly Val Ala Lys Ala Val Asp Phe Val Pro Val Glu Ser Met Glu Thr
1               5                   10                  15

Thr Met Arg Ser Pro Val Phe Thr Asp Asn Ser Ser Pro Pro Ala Val
            20                  25                  30

Pro Gln Thr Phe Gln Val Ala His Leu His Ala Pro Thr Gly Ser Gly
            35                  40                  45

Lys Ser Thr Lys Val Pro Ala Ala Tyr Ala Ala Gln Gly Tyr Lys Val
            50                  55                  60

Leu Val Leu Asn Pro Ser Val Ala Ala Thr Leu Gly Phe Gly Ala Tyr
65                  70                  75                  80

Met Ser Lys Ala His Gly Val Asp Pro Asn Ile Arg Thr Gly Val Arg
                85                  90                  95

Thr Ile Thr Thr Gly Ala Pro Ile Thr Tyr Ser Thr Tyr Gly Lys Phe
            100                 105                 110

Leu Ala Asp Gly Gly Cys Ser Gly Gly Ala Tyr Asp Ile Ile Ile Cys
            115                 120                 125

Asp Glu Cys His Ser Ile Asp Ser Thr Ser Ile Leu Gly Ile Gly Thr
        130                 135                 140

Val Leu Asp Gln Ala Glu Thr Ala Gly Ala Arg Leu Val Val Leu Ala
145                 150                 155                 160

Thr Ala Thr Pro Pro Gly Ser Val Thr Val Pro His Pro Asn Ile Glu
                165                 170                 175

Glu Val Ala Leu Ser Ser Thr Gly Glu Ile Pro Phe Tyr Gly Lys Ala
            180                 185                 190

Ile Pro Ile Glu Val Ile Lys Gly Gly Arg His Leu Ile Phe Cys His
            195                 200                 205

Ser Lys Lys Lys Cys Asp Glu Leu Ala Ala Lys Leu Ser Gly Phe Gly
        210                 215                 220

Ile Asn Ala Val Ala Tyr Tyr Arg Gly Leu Asp Val Ser Val Ile Pro
225                 230                 235                 240

```
Thr Ser Gly Asp Val Val Val Val Ala Thr Asp Ala Leu Met Thr Gly
              245             250             255

Phe Thr Gly Asp Phe Asp Ser Val Ile Asp Cys Asn Thr Cys Val Thr
              260             265             270

Gln Thr Val Asp Phe Ser
              275
```

<210> 3

<211> 14

<212> PRT

<213> hepatitis C virus


<220>

<221>  MISC_FEATURE

<222>  (7)..(7)

<223>  Xaa may be Val or Ala


<220>

<221>  MISC_FEATURE

<222>  (4)..(4)

<223>  Xaa may be Ile, Val or Thr


<400> 3

```
Ala Thr Cys Xaa Asn Gly Xaa Cys Trp Thr Val Tyr His Gly
1               5               10
```

<210> 4

<211> 9

<212> PRT

<213> hepatitis C virus


<220>

<221>  MISC_FEATURE

<222>  (2)..(2)

<223>  Xaa may be Ile, Val or Thr

```
<220>

<221>  MISC_FEATURE

<222>  (5)..(5)

<223>  Xaa may be Val or Ala


<400>  4

Cys Xaa Asn Gly Xaa Cys Trp Thr Val
1               5
```